Europäisches Patentamt

European Patent Office

Office européen des brevets

(19)

(11) Veröffentlichungsnummer: **0 086 862**
**B1**

(12) **EUROPÄISCHE PATENTSCHRIFT**

(45) Veröffentlichungstag der Patentschrift:
16.09.87

(51) Int. Cl.⁴: **A 61 F 5/00,** A 61 M 29/02

(21) Anmeldenummer: **82101703.5**

(22) Anmeldetag: **04.03.82**

(54) Vorrichtung zur Verkleinerung des Magenreservoirs mit einem intragastral applizierbaren Kissen.

(30) Priorität: **19.02.82 DE 3206118**

(43) Veröffentlichungstag der Anmeldung:
**31.08.83 Patentblatt 83/35**

(45) Bekanntmachung des Hinweises auf die Patenterteilung:
**16.09.87 Patentblatt 87/38**

(84) Benannte Vertragsstaaten:
**AT BE CH DE FR GB IT LI LU NL SE**

(56) Entgegenhaltungen:
**DE - A - 2 822 925**
**US - A - 3 834 394**
**US - A - 4 133 315**

(73) Patentinhaber: **Sir Huckleberry Assurance Services Limited, 23 Great Castle Street, London WIN 8 NQ (GB)**

(72) Erfinder: **Frimberger, Ferdinand, Dr.,**
**Josef-Kösel-Weg 10, D-8960 Kempten/Allgäu (DE)**
Erfinder: **Frimberger, Eckart, Dr., Tristanstrasse 12,**
**D-8000 München 40 (DE)**

(74) Vertreter: **Charrier, Rolf, Dipl.-Ing.,**
**Postfach 260 Rehlingenstrasse 8,**
**D-8900 Augsburg 31 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft eine Vorrichtung zum Verkleinern des Magenreservoirs mittels eines intragastral applizierbaren Kissens gemäss dem Oberbegriff des Anspruchs 1.

Übergewichtigkeit (Adipositas, Obesitas) kann als eine wesentliche Zivilisationskrankheit angesehen werden, sie ist auf jeden Fall einer der bedeutensten krankmachenden Faktoren der Gegenwart. Die Adipositas ist die Folge einer den Kalorienverbrauch übersteigenden Kalorienzufuhr. Herkömmlich erfolgt die Behandlung durch Verringerung der Kalorienzufuhr mittels Reduktionskost oder Abnahmediät. Durch Einnahme von Appetitzüglern oder durch operative Verkleinerung des Magnes kann dem Adipösen das Einhalten der Reduktionskost erleichtert werden. Verbreitet sind auch andere chirurgische Verfahren, wie Jejunoileostomie (Umgehung von Leerdarm und Krummdarm) und eine Umgehung des Magens selbst. Abgesehen davon, dass diese operativen Verfahren relativ aufwendig sind, können Komplikationen auftreten. Es sind sogar Todesfälle als Folge solcher Massnahmen beschrieben worden. Ein weiteres halboperatives Verfahren ist die sogenannte Kieferverdrahtung, bei der die Aufnahme von Nahrung erschwert wird. Auch hier sind Risiken zu erwarten.

Zur Überwindung dieser Probleme hat Hennig eine Vorrichtung der eingangs genannten Art beschrieben (DE-A-2 822 925, Inn. Med., 6 (1979), S. 149 bis 152). Hennig hat mittels eines Magenschlauchs oder auf endoskopischem Wege ein birnenförmiges doppelwandiges aus Gummihüllen bestehenden Ballon in die Magenhöhle eingeführt. Zwischen den beiden Ballonen befindet sich eine wässrige Lösung von Methylenblau. Nach der Plazierung wird mittels eines Teflonkatheters das innenliegende Kissen aufgeblasen und ebenfalls eine wässrige Lösung von Methylenblau injiziert. Nach Füllung des inneren Ballons wird der Teflonkatheter zurückgezogen. Ein Einwegeventil sowie ein gummielastisches Ventil aufgrund Eigenelastizität verschliessen die beiden Ballone im wesentlichen dicht nach aussen. Bei undichtem Ventilverschluss oder bei Perforation der Ballonhülle kommt es innerhalb von 3 bis 4 h zu einer intensiven Blaufärbung des Urins.

Bei Probanden wurde eine erhebliche Gewichtsreduktion festgestellt. Die Gewichtsreduktion beruht offensichtlich darauf, dass das Magenreservoir verkleinert wird und daher bei der Nahrungsaufnahme deutlich früher ein Völlegefühl bei dem Probanden auftritt. Untersuchungen von E. Frimberger, W. Kühner, J. Weingart, R. Ottenjahn (Münch. med. Wschr., 124 (1982)2, S. 39–40) haben die Ergebnisse von Hennig insofern bestätigen können, als durch einen intragastralen Ballon die Reduktion des Körpergewichts tatsächlich erleichtert wird. Frimberger verwendete ein dünnwandiges Kissen mit 0,06 mm Wandstärke. Durch die vorgenommene Verringerung der Wandstärke kann es leichter zum Luftaustritt durch die Kissenwand kommen, was mittels einer Beschichtung mit Silikonöl verzögert wurde. Auch Nieben und Harboe (The Lancet, (23. Januar 1982), S. 198/199) haben einen intragastral applizierbaren Ballon verwendet, wobei zum Applizieren wie bei Hennig zwei ineinander geführte Sonden verwendet wurden.

Bei derartigen intragastral applizierbaren Ballonen treten sowohl beim Entfernen des Ballons aus dem Magen als auch beim Applizieren Probleme auf.

Der doppelwandige Ballon nach Hennig wird mit der Diathermie-Schlinge nach Deyle-Seuberth unter gastroskopischer Sicht nach vorausgegangener Perforation mit einer endoskopisch eingeführten Injektionsnadel extrahiert. D. h. die Extraktion erfolgt ebenfalls oral und ist ziemlich aufwendig und auch komplikationsverdächtig. Platzt ein solcher Ballon, so kann er aufgrund seines Raumbedarfes nicht ohne weiteres über den Darm abgehen, es kann zu Darmverstopfungen kommen, die operativ beseitigt werden müssten.

Dieses Problem wird durch das dünnwandige Kissen nach Frimberger et al. und den Ballon nach Nieben und Harboe überwunden. Beide Ballon-Typen sind derart dünnwandig, dass sie im wesentlichen komplikationslos nach Platzen durch den Darm abgehen können und mit dem Stuhl ausgeschieden werden. Es kann sogar auftreten, dass der Patient oder Proband das Abgehen des Ballons nicht merkt.

Jedoch ist das Applizieren des Ballons problematisch. Beim Einführen des Applikators mit dem Ballon durch Pharynx (Rachen) und Ösophagus (Speiseröhre) kann es auch einem sehr versierten Operateur passieren, dass der Ballon und der Applikator versehentlich in die Trachea (Luftröhre) eingeführt werden. Bei dem dann sofort auftretenden akuten Erstickungsanfall muss der Applikator mit dem Ballon sofort aus der Trachea zurückgezogen werden. Bei der von Hennig erläuterten Verbindung zwischen Applikator und Ballon bei der lediglich ein Schlauch durch eine den Ballonrand umgebende Manschette geführt ist, kann es dabei sehr leicht passieren, dass der Schlauch aus dem Ventil herausgezogen wird, also der Ballon vom Applikator gelöst wird und in der Trachea zurückbleibt. Dies kann lebensgefährliche Komplikationen zur Folge haben, wenn nicht sofort ein erfahrener Arzt mit dem erforderlichen Instrumentarium zur Verfügung steht, um den Ballon aus der Trachea zu entfernen. Weder bei Hennig noch bei den anderen Verfassern sind Hinweise auf die Überwindung dieses Problems zu entnehmen. Weiter ermöglicht das von Hennig verwendete Einwegeventil, bei dem es sich offenbar um ein Kugelventil handelt, lediglich das Füllen des Ballons mit dem Fluid, wobei dann, wenn der Ballon bis zu einem Füllungsgrad gefüllt ist, bei dem der Proband ein unbequemes Gefühl bekommt, ein Entleeren nicht mehr möglich ist. In einem solchen Fall muss bei der Vorrichtung nach Hennig das Kissen in der vorstehend erläuterten Weise entfernt werden.

Es ist Aufgabe der Erfindung eine Vorrichtung der eingangs genannten Art so auszubilden, dass das Kissen leicht applizierbar ist und nicht die Gefahr des unbeabsichtigten Ablösens des Kissens vom Applikator besteht.

Die Aufgabe wird durch die kennzeichnenden Merkmale des Anspruchs 1 und/oder des Anspruchs 8 gelöst.

Die Erfindung wird durch die Merkmale der abhängigen Ansprüche weitergebildet.

Dabei wird unter dem Begriff Kissen ein Hohlbehälter verstanden, der durch das Fluid ohne wesentliche Dehnung seiner Wandungen in seine vorhergehende Form gebracht wird. Insbesondere wird durch die Erfindung eine komplikationsfreie Applizierung für den Patienten/Probanden möglich und wird eine komplikationsfreie Anwendung des Kissens selbst erreicht. Schliesslich ist eine ambulante Applizierung möglich. Auch das Entfernen des Kissens ist auf einfache Weise möglich. Wesentlich ist, dass für das Plazieren des Kissens im Magen eine sichere und stabile Verbindung zwischen Applikator und Kissen erreicht ist, obwohl, anders als Hennig lediglich ein einwandiges Kissen dünner Wandstärke verwendet ist. Wesentlich ist ferner, dass nach dem Füllen im Magen das Kissen im gleichen Arbeitsgang auch wieder ganz oder teilweise entleert werden kann, wenn bei dem Patienten Beschwerden auftreten sollten. Wenn das plazierte Kissen undicht wird, kann es auf einfache Weise durch den Darm mit dem Stuhl abgehen. Es besteht nicht die Gefahr, dass das Kissen an einer Engstelle im Darmtrakt (z.B. beim Übergang von Dünn- auf Dickdarm, der Ileozökalklappe) hängen bleibt. Wenn eine Füllung mit Wasser verwendet wird, liegt das Kissen nahe dem Pylorus (Pförtner), während es bei Luftfüllung nahe dem Fundus liegt, insbesondere wenn der Magen gefüllt ist.

Das Kissen wird beim Plazieren nicht gezielt im Pylorus- oder im Fundus-Bereich lokalisiert, sondern nimmt von selbst die entsprechende Lage ein. Bei Wasserfüllung sinkt es aufgrund seines Gewichtes an die tiefste Stelle des Magens ab, bei Gas- oder Luftfüllung schwimmt es auf dem Magensaft, bzw. dem Mageninhalt.

Wesentlich für die erfindungsgemässe Vorrichtung ist jedoch, dass das Applizieren gefahrlos erfolgen kann, da die Vorrichtung selbst dann, wenn sie versehentlich in die Trachea eingeführt wird, schnell und sicher wieder herausgezogen werden kann, ohne dass die Gefahr besteht, dass sich das Kissen vom Applikator löst. Insbesondere durch Falten über den benachbarten Abschnitt des Applikators oder durch Umgeben mit einer zusätzlichen Hülle, kann ein – auch psychologisch – unangenehmes Gefühl für den Patienten beim Einführen vermieden werden. Bei einem dünnwandigen Kissen hat das Undichtwerden des Kissens nach bestimmter Tragedauer den nicht unerwünschten Effekt, dass das Kissen schnell und möglicherweise vom Patienten unbemerkt abgeht. Das gleiche kann auftreten, wenn bei einer Luftfüllung aufgrund der Luftdurchlässigkeit des Materials des Kissens diese entweicht und das Kissen dann abgeht. Das Entfernen des Kissens aus dem Magen kann auch auf einfache Weise dadurch erreicht werden, dass dessen Wandung mittels eines endoskopisch eingeführten Werkzeugs aufgerissen wird. Dies könnte auch dadurch erreicht werden, dass der Patient ein die Wandung des Kissens zersetzendes, im übrigen selbstverständlich magenfreundliches Mittel einnimmt.

Schliesslich ist noch zu erwähnen, dass das abgehende Kissen selbst dann, wenn es infolge noch zu grosser Füllung im Dünndarm stecken bleibt, mittels bekannter und praktisch risikofreier transabdomineller Feinnadel-Punktion perforiert und damit von seiner Füllung befreit werden kann.

Schliesslich kann, wenn es erwünscht ist, dass der Patient feststellen kann, wenn das Kissen defekt (undicht) oder geplatzt ist, dem Kissen eine geeignete Menge eines bioneutralen und unschädlichen Farbstoffes in flüssiger oder fester Form bereits beim Herstellen der Vorrichtung zugefügt werden. Der Farbstoff, wie beispielsweise Methylenblau, tritt aus dem undichten Kissen aus und wird mit dem dann blaugefärbten Urin ausgeschieden. Auch ein keimtötendes Mittel kann beigefügt werden.

Es sei noch erwähnt, dass noch über ein intragastral appliziertes Kissen berichtet worden ist, bei dem der Applikator nicht entfernt ist, vielmehr ein mit dem Kissen verbundener Schlauch aus dem Mund des Patienten herausragt und dort mittels eines Stöpsels verschlossen ist (vgl. European Rubber Journal, (Juli 1981), S. 37). Diese Vorrichtung wird offenbar vom Patienten verschluckt und dann mit Luft und/oder Wasser gefüllt, wobei weiter ein röntgenologisches Kontrastmittel enthalten sein kann. Zur Entfernung wird das Kissen mittels dem Schlauch wieder oral entfernt. Die eingangs erwähnten Probleme bei von einem Applikator lösbaren Kissen treten bei einer derartigen Vorrichtung nicht auf.

Die Erfindung wird anhand der in der Zeichnung dargestellten Ausführungsbeispiele näher erläutert.

Es zeigen:

Fig. 1 schematisch und im Schnitt eine erfindungsgemässe Vorrichtung im applizierbaren Zustand,

Fig. 2 das Kissen der Vorrichtung während des Füllens mit Fluid,

Fig. 3 schematisch das applizierte, fluidgefüllte Kissen,

Fig. 4 Einzelheiten der Zuordnung von Applikator und Verschlusskörper,

Fig. 5 schematisch eine andere Ausführungsform der Eingriffsteile,

Fig. 6 schematisch die ausser Eingriff befindlichen Eingriffsteile gemäss Fig. 5,

Fig. 7 schematisch eine andere Ausführungsform der erfindungsgemässen Vorrichtung,

Fig. 8 bis 11 weitere Ausführungsformen der Eingriffsteile.

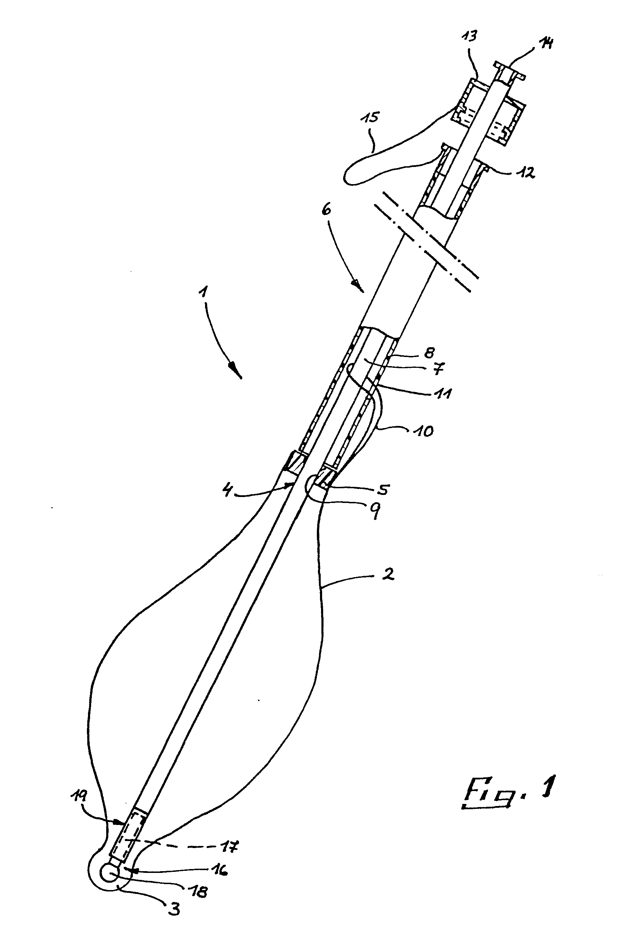

Fig. 1 zeigt schematisch eine erste Ausführungsform einer erfindungsgemässen Vorrichtung 1.

Sie besteht aus einem intragastral, d.h. im Magen, applizierbaren Kissen 2 aus einem weichen, nichttoxischen und durch die Magensäfte zumindest nur wenig angreifbaren Wirkstoff, wie einem Kautschuk, Polyäthylen (PE), Polytetrafluoräthylen (PTFE, z.B. Teflon), Polyvinylchlorid (PVC), Silikon oder dergleichen. Das Kissen 2 hat ein Fassungsvolumen von etwa 100 bis 1000 ml. Es können mehrere Kissen 2 unterschiedlicher abgestufter Grössen vorgesehen sein. Das Kissen 2 gemäss Fig. 1 weist an seinem vorderen Ende eine kleine nasenartige Ausstülpung 3, sowie am gegenüberliegenden hinteren Ende eine kleine Öffnung 4 auf. In die Öffnung 4 ist ein Ring 5 eingesetzt, der aus einem Kautschuk (Gummi) oder Kunststoff bestehen kann, wie das noch näher erläutert wird. Die Vorrichtung 1 weist weiter einen mit dem Kissen 2 an dessen die Öffnung 4 aufweisenden Ende fest, aber lösbar verbundenen Applikator 6 auf.

Der Applikator 6 besteht aus nachgiebigem biegeelastischem Werkstoff und dient dazu das Kissen 7 peroral durch Pharynx (Rachen) und Ösophagus (Speiseröhre) in den Ventrikulus (Magen) zu applizieren. Demgemäss weisen die beiden miteinander verbundenen Teile der Vorrichtung 1, nämlich das Kissen 2 und der Applikator 6 eine Gesamtlänge in der Grössenordnung von etwa 70 cm auf.

Der Applikator 6 besteht aus zwei ineinander angeordneten und gegeneinander führbaren Sonden, nämlich einer Innensonde 7 und einer Aussensonde 8. Die Aussensonde 8 besitzt einen Aussendurchmesser in der Grössenordnung von 5 mm und die Innensonde 7 besitzt einen Aussendurchmesser in der Grössenordnung von 3 mm bei einem Innendurchmesser in der Grössenordnung von etwa 2,6 mm.

Die als Schläuche ausgebildeten Sonden 7 und 8 bestehen aus einem nichttoxischen elastischen Werkstoff, wie einem Polyäthylen (PE), Polyvinylchlorid (PVC) oder Polytetrafluoräthylen (PTFE). Zweckmässigerweise weisen Innen- und Aussensonde 7, 8 eine Röntgenstrahlendurchlässigkeit auf, die kleiner als die des umgebenden Gewebes ist, derart, dass deren Lage am Röntgenschirm verfolgt werden kann.

Weiter können der Ring 5 sowie ein noch später zu erläuternder Verschlusskörper 16 ebenfalls eine Röntgenstrahlendurchlässigkeit besitzen, die kleiner als die des umgebenden Gewebes ist, derart, dass deren Lage am Röntgenschirm verfolgt werden kann. Dies ist dann von Vorteil, wenn bei Abgehen des Kissens dessen Lage im Darmtrakt festgestellt, bzw. überwacht werden soll.

Über die Innensonde ist nach intragastraler Applizierung des Kissens 2 ein Fluid, wie Wasser und/oder Luft einführbar derart, dass das Kissen 2 im erwünschten Masse gefüllt wird. Zur Füllung kann auch ein anderes Fluid verwendet sein, das jedoch nichttoxisch und magenverträglich sein muss. Das Fluid kann Indikatoren aufweisen, mittels denen das Undichtwerden und damit das Entweichen des Fluids oder der Abgang des Kissens 2 festgestellt werden können. Beispielsweise kann das Kissen 2 bereits Methylenblau fertigungsseitig enthalten, das bei Austritt mit dem Fluid in den Magenraum eine Blaufärbung des Urins verursacht.

Eine einfache feste aber lösbare Verbindung zwischen Applikator 6 und Kissen 2 besteht darin, dass der Ring 5 zumindest im Bereich seiner Mittelöffnung 9 aus einem elastischen Werkstoff besteht, dessen Innendurchmesser deutlich kleiner als der Aussendurchmesser der Innensonde 7 ist. Bei in das Kissen 2 eingeführtem Zustand umschliesst der Ring 5 die Innensonde 7. Nach dem Herausziehen der Innensonde 7 aus dem Kissen 2 durch den Ring 5 zum Entfernen des Applikators 6 vom Kissen 2, legen sich die Wände des Rings 5 um die Mittelöffnung 9 dicht gegeneinander an und verschliessen so das Kissen 2. Dies wird beispielsweise dadurch erreicht, dass die Mittelöffnung 9 schlitzartig ausgebildet ist. Dieser dichte Verschluss 9 muss zumindest bis zu einem vorgegebenen Druck des Fluids im Kissen 2 auf dem Ring 5 aufrechterhalten bleiben. Es ist jedoch nicht notwendig, dass die Dichtheit bis zur Platzgrenze des Kissens 2 beibehalten ist.

Beim Einführen einer solchen Vorrichtung durch den Ösophagus kann es auch einem geübten Arzt passieren, dass die Vorrichtung versehentlich in die Trachea (Luftröhre) eingeführt wird. Bei dem dann sofort auftretenden akuten Erstickungsanfall muss der Applikator mit dem Kissen sofort aus der Trachea zurückgezogen werden. Würde sich in einer derartigen Situation das Kissen vom Applikator lösen und in der Trachea zurückbleiben, können gefährliche, lebensbedrohende Komplikationen auftreten, wenn nicht sofort ein erfahrener Arzt mit dem erforderlichen Instrumentarium eingreift.

Zur Überwindung dieses Problems weist die erfindungsgemässe Vorrichtung 1 eine Sicherung dergestalt auf, dass Kissen 2 und Applikator 6 solange sicher in Eingriff bleiben, bis durch eine bewusste Bewegung der Innensonde ein Lösen des Eingriffs erreicht und damit eine Trennung von Applikator 6 und Kissen 2 ermöglicht wird.

Bei dem in Fig. 1 dargestellten Ausführungsbeispiel ist am Kissen 2 im Bereich dessen Rings 5 eine Fadenschlaufe 10 befestigt und durch eine seitliche Öffnung 11 am proximalen Endabschnitt der Aussensonde 8 in das Innere der Aussensonde 8 derart geführt, dass die Schlaufe von der Innensonde 7 durchsetzt ist.

Applikator 6 und Kissen 2 sind daher nur dann voneinander lösbar, wenn die Innensonde 7 durch die Fadenschlaufe 10 hindurchgezogen worden ist.

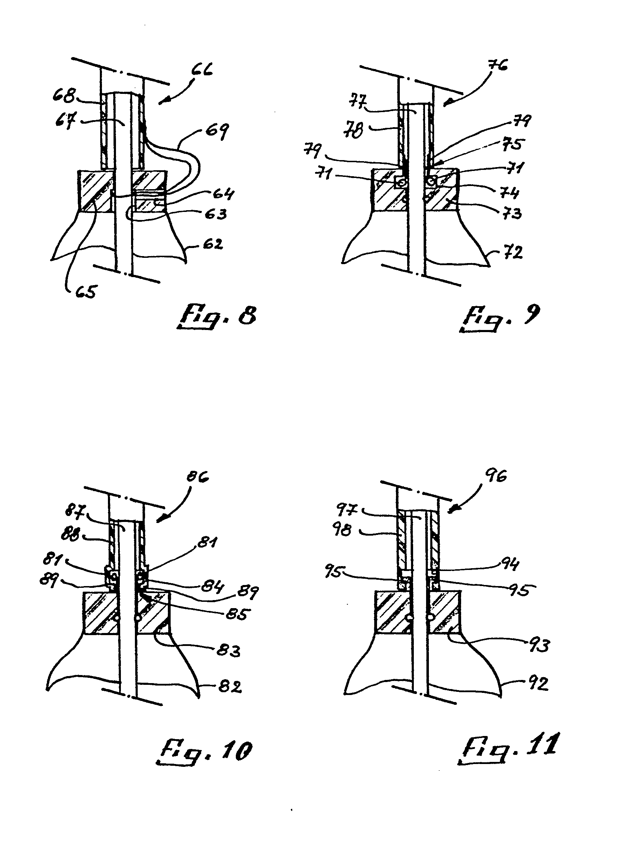

Gemäss Fig. 8 kann die Sicherung auch so ausgebildet sein, dass eine Fadenschlaufe 69 an der Aussensonde 68 des Applikators 66 befestigt ist und durch eine Queröffnung 64 im Ring 65 im Kissen 62 in die Mittelöffnung 63 des Rings 65 eingeführt ist, wobei die Innensonde 67 des Applikators 66 die Fadenschlaufe 69 durchsetzt. Auch hier sind Applikator 66 und Kissen 62 daher nur dann voneinander lösbar, wenn die Innensonde 67 durch die Fadenschlaufe 69 hindurchgezogen worden ist.

Um zu verhindern, dass versehentlich nur die Innensonde 7 bewegt, insbesondere herausgezogen wird, sind weiter Innensonde 7 und Aussensonde 8 am distalen Ende, d.h. dem Ende, das beim Applizieren aus dem Mund des Patienten herausragt, fest aber lösbar miteinander verbunden. Diese Verbindung erfolgt zweckmässigerweise über eine Schnellkupplung wie eine Luer-Lock-Verbindung oder ein Bajonettverschluss. Bei dem Ausführungsbeispiel gemäss Fig. 1 trägt das distale Ende der Aussensonde 8 ein inneres Kupplungsteil 12, das von einem äusseren Kupplungsteil 13 übergreifbar ist, das an der Innensonde 7 befestigt ist. Weiter zeigt Fig. 1 das distale Ende 14 der Innensonde 7, das zum Einfüllen des Fluids in das intragastral applizierten Kissen 2 dient und zweckmässigerweise zur Ankopplung an eine entsprechende Fülleinrichtung ausgebildet ist.

Um den Applikator 6 nach Lösen des Eingriffs zwischen Applikator 6 und Kissen 2 möglichst schnell aus dem Ösophagus entfernen zu können, ist eine Erfassungseinrichtung vorgesehen, mittels der erfasst werden kann, dass die Innensonde 7 das Kissen 2 verlassen hat und gegebenenfalls durch die Fadenschleife 10 hindurchgetreten ist. Bei dem Ausführungsbeispiel gemäss Fig. 1 sind die Innensonde 7 und die Aussensonde 8 am distalen Ende, hier den beiden Kupplungsteilen 12 und 13 durch eine verformbare, jedoch in ihrer Längserstreckung nicht dehnbare, Verbindung 15 miteinander verbunden, deren Länge mindestens dem Bewegungsweg der Innensonde 7 zwischen dem Eingriffszustand der Kupplungsteile 12 und 13 und dem sicheren Hindurchtreten durch die Fadenschleife 10 entspricht. Ist also die Verbindung 15 zwischen den Kupplungsteilen 12 und 13 gestreckt, so sind Applikator 6 und Kissen 2 ausser Eingriff und kann der Applikator 6 dann insgesamt schnell aus dem Ösophagus entfernt werden.

Wie erwähnt, kann es genügen, dass der Ring 5 nach Entfernen der Innensonde 7 einen dichten Abschluss erreicht. Ein solcher dichter Abschluss kann noch dadurch verbessert werden, dass die Mittelöffnung 9 des Rings 5 mit einem Silikonöl oder einem vergleichbaren Dichtungsmittel beschichtet ist.

Der Ring 5 kann aber auch aus einem im wesentlichen unverformbaren oder nur wenig verformbaren Werkstoff bestehen, derart, dass die Mittelöffnung 9 nach Entfernen der Innensonde 7 nicht vollständig verschlossen wird.

Die erwähnte Verschlussmöglichkeit hat jedoch einen weiteren Nachteil. Dann, wenn die Vorrichtung als applizierbare fertigverpackte Einheit verwendet wird, bei der die Innensonde des Applikators die Mittelöffnung durchsetzt, kann durch die Lagerung die Innensonde deformiert werden und/oder kann der Schlitzverschluss aufgrund Materialermüdung oder dgl. ebenfalls bleibend verformt werden, derart, dass beim Füllen und nach dem Entfernen der Innensonde ein dichtes Verschliessen des Kissens nicht mehr gewährleistet werden kann, also die Verwendung der Vorrichtung nicht mehr als sicher angesehen werden müsste.

Es wurde daher bereits von Hennig vorgeschlagen, ein verschliessbares Ventil, nämlich ein Einwegeventil, vermutlich ein Kugelventil, zu verwenden. Ein Einwegeventil hat den grundsätzlichen Nachteil, dass lediglich der Betrieb in einer Richtung, hier zum Füllen des Kissens möglich ist. Wenn nun das Kissen soweit gefüllt ist, dass für den Patienten ein Missbehagen oder ein ungutes Gefühl auftritt, kann bei dem bekannten Ventil das Kissen nicht mehr entleert werden. Vielmehr muss das Kissen in der erläuterten Weise aufgerissen und aus dem Magen wieder entfernt werden. Da dies unter Umständen beim gleichen Patienten mehrfach auftreten könnte, ist diese Vorgehensweise für die ambulante Behandlung ungeeignet.

Zur Überwindung dieses Problems wird daher erfindungsgemäss beim Entfernen der Innensonde 7 ein Verschlusskörper 16, wie ein Stöpsel aus einem verträglichem Werkstoff in die Mittelöffnung 9 eingesetzt. Der Verschlusskörper 16 weist einen Halsabschnitt 17 und einen verdickten, hier kugelförmigen Abschnitt 18 auf, wobei der Halsabschnitt 17 am proximalen Ende der Innensonde 7 angebracht ist und beim Entfernen der Innensonde 7 durch die Mittelöffnung 9 durch Anlage des verdickten Abschnitts 18 gegen den Ring 5 von der Innensonde 7 gelöst und in die Mittelöffnung 9 zum dichten Verschliessen des Kissens 7 eingesetzt wird.

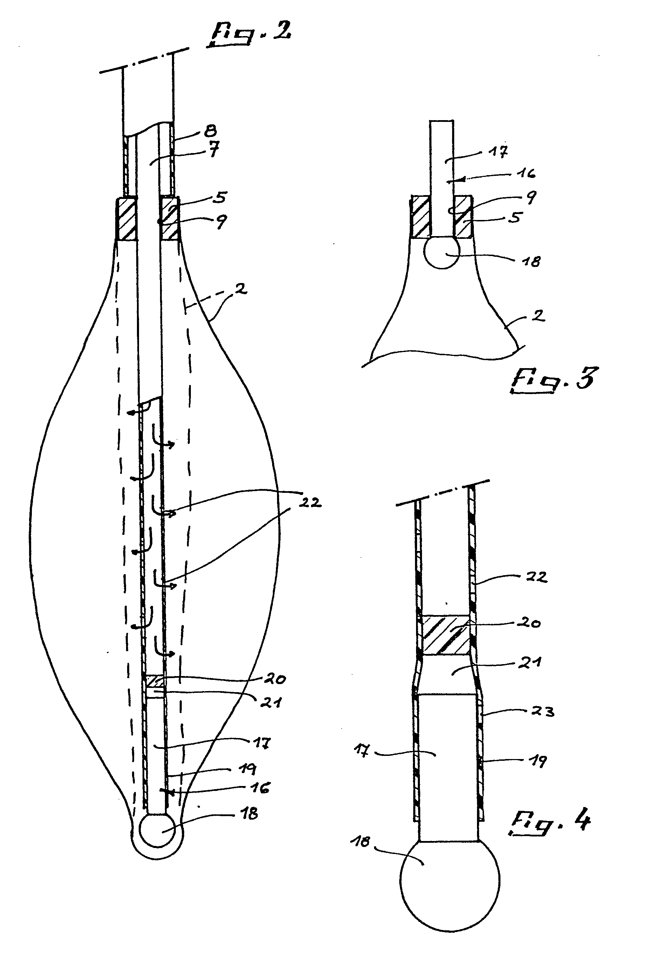

Dies wird anhand der Fig. 2 bis 4 näher erläutert.

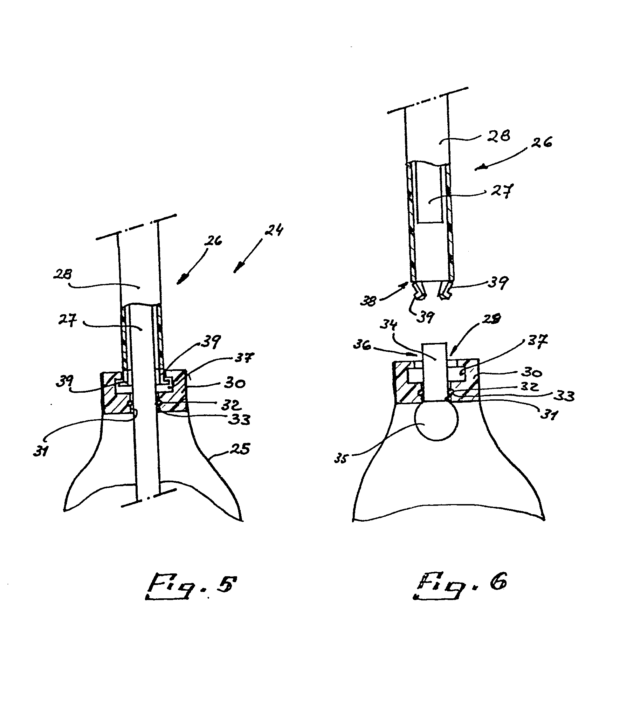

Am proximalen Ende 19 der Innensonde 7 ist mittels einer Zwischenwand 20 ein Hohlraum 21 vom übrigen Innenbereich der Innensonde 7 abgetrennt. In den Hohlraum 21 ist der Verschlusskörper 16 mit seinem Halsabschnitt 17 eingesetzt. Der Aussendurchmesser des Halsabschnittes 17 entspricht dabei mindestens dem Innendurchmesser des Hohlraums 21, wobei, wie das Fig. 4 zeigt, der Innendurchmesser des Hohlraums 21 etwas grösser als der Innendurchmesser der übrigen Innensonde 7 sein kann, und zwar um etwa 0,2 bis 1,0 mm. Die Innensonde 7 kann in diesem Bereich aber auch aus elastisch verformbarem Werkstoff bestehen.

Um das Fluid in das Kissen 7 einfüllen zu können, weist die Innensonde 7 in dem sich an die Zwischenwand 20 anschliessenden Bereich Perforationen 22 auf, durch die das vom distalen Ende 14 zugeführte Fluid in das Kissen 2 ausströmen kann. Die Innensonde 7 wird nach der Füllung des Kissens 2 aus diesem herausgezogen. Das Ausmass der Füllung wird im allgemeinen durch die Aussagen des Patienten beim Füllen bestimmt, kann aber auch durch z.B. Betrachtung auf dem Röntgenschirm oder Vorsehen von geeigneten Messinstrumenten an der distalen Öffnung 14 der Innensonde 7 überwacht werden.

Fig. 2 zeigt in Strichlinien das Kissen 2 ohne Fluidfüllung, d.h. in dem Zustand in dem es intragastral appliziert wird, und in Vollinien das zumindest teilweise mit Fluid gefüllte Kissen 2.

Ist das Kissen 2 mit dem Fluid im erwünschten Ausmass gefüllt, wird die Innensonde 7 mit dem Verschlusskörper 16 durch den Ring 15 hindurch nach oben herausgezogen, wobei der verdickte Abschnitt 18 des Verschlusskörpers 16 zur Anlage an den Ring 5 kommt und dadurch der Verschlusskörper 16 aus dem Hohlraum 21 herausgezogen wird und mit seinem Halsabschnitt 17 in der Mittelöffnung 9 des Rings 5 zum dichten Verschliessen des Kissens 2 verbleibt.

Fig. 4 zeigt, dass auch in der Wand des Hohlraums 21 eine Perforation 23 ausgebildet sein kann, die hier bei in den Hohlraum 21 eingesetztem Halsabschnitt 17 des Verschlusskörpers 16 von diesem verschlossen ist. Wenn beim Herausziehen der Innensonde 7 der verdickte Abschnitt 18 zur Anlage an den Ring 5 kommt, wird nach geringem Hubweg die Perforation 23 freigelegt, wodurch zwischen dem zur Zwischenwand 20 weisenden Ende des Halsabschnitts 17 und der Zwischenwand 20 kein saugender Unterdruck entstehen kann, wodurch wiederum das Abziehen der Innensonde 7 bzw. dessen proximalem Ende 19 von dem Halsabschnitt 17 erleichtert wird. Die Perforation 23 kann jedoch auch ganz oder teilweise freiliegen.

Wie erwähnt, ist die sichere, jedoch lösbare Verbindung zwischen Applikator 6 und Kissen 2 von wesentlicher Bedeutung.

In den Fig. 5 und 6 ist eine andere grundsätzliche Ausführungsform einer erfindungsgemässen Vorrichtung 24 dargestellt.

Das dem Kissen 2 entsprechende Kissen 25 ist mit dem dem Applikator 6 entsprechenden Applikator 26, der eine Innensonde 27 und eine Aussensonde 28 aufweist, in anderer Weise verbunden. Das Kissen 25 weist an seiner Öffnung 29 wieder einen Ring 30 auf, der hier aus im wesentlichen unverformbarem Werkstoff, wie einem Kunststoff besteht. In seiner Mittelöffnung 31 weist der Ring 30 zunächst eine dem Kissen 25 zugewandte elastisch verformbare Dichtung auf, die durch einen in eine Ringnut 32 eingelegten O-Ring 33 aus elastischem Werkstoff gebildet ist, dessen Innendurchmesser kleiner als der Aussendurchmesser der Innensonde 27 und auch kleiner als der Aussendurchmesser des Halsabschnitts 34 des Verschlusskörpers 36 ist, der ebenfalls einen verdickten Abschnitt 35 aufweist und in der gleichen Weise wie der Verschlusskörper 16 gemäss den Fig. 1 bis 4 eingesetzt werden kann (im Einzelnen nicht dargestellt).

Im zum Applikator 26 weisenden Bereich der Mittelöffnung 29 weist der Ring 30 eine weitere grössere Ringnut 37 auf, die ein Eingriffsteil der sicheren Verbindung zwischen Applikator 26 und Kissen 25 bildet.

In der proximalen Stirnseite 38 der Aussensonde 28 sind hier Hakenelemente 39 angeformt, die zum Eingriff in die Ringnut 37 ausgebildet sind. Die Hakenelemente 39 können eine radial nach innen gerichtete Vorspannung derart aufweisen, dass sie ohne Gegenkraft nicht in Eingriff mit der Ringnut 37 kommen können, wie das in Fig. 6 dargestellt ist. Ist jedoch die Innensonde 27 an den Hakenelementen 39 vorbeigeführt, wie in Fig. 5 dargestellt, sind die Hakenelemente 39 radial nach aussen gedrückt und greifen dabei in die Ringnut 37 im Ring 30 ein und werden dort durch die Innensonde 27 in Lage gehalten.

Wesentlich ist also, dass durch die in das Kissen 25 eingeführte Innensonde 27 die Hakenelemente 39 in die Ringnut 37 sicher eingreifen und dass, wie bei dem Ausführungsbeispiel gemäss Fig. 1 der Eingriff zwischen dem Applikator 26 und dem Kissen 25 nur dadurch lösbar ist, dass die Innensonde 27 an den Hakenelementen 39 vorbei in Richtung auf das distale Ende aus dem Kissen 25 herausgezogen ist, da dann, z.B. aufgrund der radial wirkenden Vorspannung, die Hakenelemente 39 sich nach innen bewegen und ausser Eingriff mit der Ringnut 37 im Ring 30 kommen.

Andere Ausführungsformen werden weiter unten mit Bezug auf die Fig. 9–11 näher erläutert.

Die Halterung des Verschlusskörpers 36 an der Innensonde 27 kann in der gleichen Weise erfolgen wie das anhand der Fig. 1 bis 3 erläutert worden ist. Am distalen Ende können Innensonde und Aussensonde 27 bzw. 28 in der gleichen Weise ausgebildet sein, wie das anhand Fig. 1 erläutert worden ist.

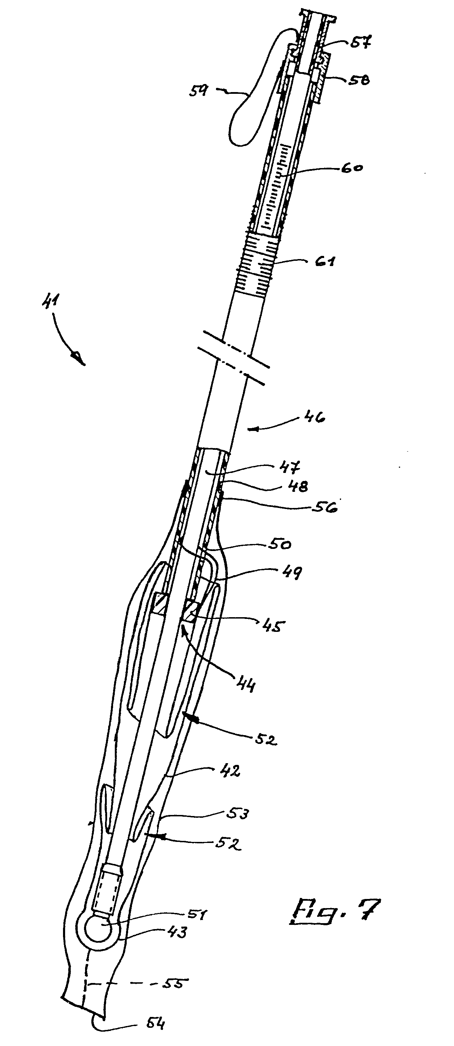

Fig. 7 zeigt eine weitere Ausführungsform der Erfindung, deren Elemente fakultativ auch bei den anderen bereits erwähnten Ausführungsformen anwendbar sind.

Die Vorrichtung 41 gemäss Fig. 7 weist ein Kissen 42 auf, das ebenfalls eine Ausstülpung 43 aufweisen kann und das in seiner Öffnung 44 einen Ring 45 enthält. Das Kissen 42 ist hier mit einem Applikator 46 aus Innensonde 47 und Aussensonde 48 in der anhand Fig. 1 erläuterten Weise fest aber lösbar verbunden, d.h. weist eine am Ring 45 befestigte Fadenschleife 49 auf, die durch eine Öffnung 50 in dem proximalen Abschnitt der Aussensonde 48 geführt ist und von der Innensonde 47 durchsetzt ist. Selbstverständlich kann auch eine andere gleichwirkende Verbindung, wie über Hakenelemente (Fig. 5, 6, 9 bis 11) vorgesehen sein.

Die Innensonde 47 trägt an ihrem proximalen Ende in der bereits erläuterten Weise einen Verschlusskörper 51 zum dichten Verschliessen des Kissens 42 bei Herausziehen der Innensonde 47. Wie dargestellt, kann das Kissen 42 zwischen dem an dem Verschlusskörper 51 liegenden, die Ausstülpung 43 aufweisenden Ende und dem am Ring 45 befestigten Ende in Falten 52 über die Innensonde 47 und/oder die Aussensonde 48 gelegt sein.

Wesentlich für diese Ausführungsform ist, dass am proximalen Abschnitt distal zur Öffnung 50 eine schlauchartige Hülle 53 an einem Ende 56 an der Aussensonde 48 befestigt ist.

Die Hülle 53 kann wie dargestellt bei applikationsbereiter Vorrichtung 41 etwas über das proximale Ende der Innensonde 47 und insbesondere über die Ausstülpung 43 des Kissens 42 hinausragen. Die Hülle 53 muss jedoch nicht über das proximale Ende der Innensonde 47 hinausragen, sie kann beispielsweise auch in Höhe des Verschlusskörpers 51 am Übergang von dessen Halsabschnitt zu dessen verdickten Abschnitt enden.

Weiter muss das proximale Ende 54 der Hülle 53 nicht notwendigerweise verschlossen sein, sondern kann, wie dargestellt, offen liegen. Die Hülle 53 bewirkt, dass das gefaltete Kissen 42 mit seiner mindestens einen Faltung 52 fixiert ist und das Kissen derart zusammengehalten ist, dass es vor dem Einführen optisch kleiner wirkt, was eine positive psychologische Wirkung auf den Patienten hat. Weiter stellt die Hülle 53 für das Applizieren einen Schutz des relativ empfindlichen Kissens 42 dar, muss jedoch beispielsweise bei dessen Füllen mit dem Fluid leicht entfernbar sein. Beim Füllen verschiebt sich das Kissen 42 zur Auffaltung der Falten 52 in axialer Richtung und schiebt sich dabei aus der Hülle 53 an dessen Ende 54 heraus. Die Hülle 53 wird dabei nach hinten verschoben und kann dabei aufgerollt werden. Sie besteht dann vorteilhaft aus einem leichter dehnbaren Werkstoff wie das Kissen. Die Hülle 53 kann jedoch auch aus widerstandsfähigerem und weniger elastischerem Werkstoff wie das Kissen 52 bestehen, wie einem Kautschuk oder Kunststoff. Sie enthält dann vorteilhaft eine in Fig. 7 schematisch dargestellte Reisslinie 55, längs der die Hülle 53 beim Füllen des Kissens 42 aufreisst. Schliesslich kann die Wandstärke der Hülle 53 von dem an der Aussensonde 58 befestigten Ende 56 bis zum proximalen Ende 54 allmählich oder stufenweise abnehmen, wodurch das Ablösen der Hülle 53 von dem sich füllenden Kissen 42 erleichtert wird. Die Hülle 53 bleibt dabei an der Aussensonde 58 und wird nach Lösen des Applikators 46 vom Kissen 42 mit ersterem wieder extrahiert.

Ähnlich wie bei dem Ausführungsbeispiel gemäss Fig. 1 sind Innensonde und Aussensonde 47 bzw. 48 an ihrem distalen Ende über Kupplungsglieder 57 bzw. 58 fest aber lösbar miteinander verbunden. Hier ist das innenliegende Kupplungsglied 57 an der Innensonde 47 befestigt, während das aussenliegende, das innenliegende Kupplungsglied 57 übergreifende Kupplungsglied 58 an der Aussensonde 48 befestigt ist. Weiter kann eine der Verbindung 15 gemäss Fig. 1 entsprechende Vorrichtung 59 vorgesehen sein.

Bei diesem Ausführungsbeispiel ist weiter wesentlich, dass mindestens eine der beiden Sonden 47, 48 ein Markierungsfeld aufweist.

Mittels eines Markierungsfeldes 60 an der Innensonde 47, kann die Ist-Lage der Innensonde 47 gegenüber der Aussensonde 48 dadurch festgestellt werden, ob und wie weit das Markierungsfeld 60 am distalen, dem Kupplungsglied 58 zugewandten Ende der Aussensonde 48 ausgetreten ist. Dadurch kann zunächst festgestellt werden, wann der Verschlusskörper 51 in den Ring 45 zum Verschliessen des Kissens 42 eingesetzt ist und kann weiter festgestellt werden, ob die Innensonde 47 den Eingriff der Eingriffteile gelöst hat.

Weiter kann auch die Aussensonde 48 zumindest an ihrem distalen Endabschnitt durchsichtig sein und zumindest eine einzige Markierung aufweisen, wobei dann die Bewegung und damit die Ist-Lage der Innensonde 47 gegenüber der Aussensonde 48 genauer beobachtet werden kann.

Darüberhinaus kann auch die Aussensonde 48 an ihrem distalen Endabschnitt ein Markierungsfeld 61 tragen. Letzteres Markierungsfeld 61 an der Aussensonde 48 hat Vorteile beim Applizieren. Mit diesem Markierungsfeld 61 auf der Aussensonde 48 kann festgestellt werden, wie weit die Vorrichtung 41 und damit das Kissen 42 den Magen erreicht und in diesen eingeführt ist und damit gefüllt werden kann. Das Markierungsfeld 61 könnte beispielsweise eine etwa 10 cm lange Farbzone im Abstandsbereich zwischen 50 und 60 cm von dem Vorderabschnitt der Vorrichtung, hier dem vordersten Ende der Ausstülpung 43 und/oder des Verschlusskörpers 51 sein, was dem durchschnittlichen Abstand zwischen Magen und Mundöffnung entspricht. Bei kleineren Patienten wird die Vorrichtung 41 bis zum Beginn des Markierungsfeldes 61, bei grösseren Patienten bis zum Ende des Markierungsfeldes 61 eingeführt. Das Markierungsfeld 61 kann anstelle oder zusätzlich zu Farbzonen auch ringförmige Markierungen tragen, wie das in Fig. 7 dargestellt ist.

Es sei erwähnt, dass die Kupplungsglieder 57 und 58 in Schraubeingriff sein können oder nach Art eines Bajonettverschlusses miteinander verbunden sein können.

Falls die Hülle 53 fest geschlossen ist, kann sie einen sterilen Schutz für das Kissen 52 bilden.

Es zeigt sich, dass die erfindungsgemässe Vorrichtung als Einmal-Einheit verwendbar ist, dass aber auch der Applikator 6 bzw. 26 bzw. 46 ohne weiteres mehrfach verwendet werden kann.

Beim intragastralen Applizieren des Kissens 2 bzw. 25 bzw. 42 wird wie folgt vorgegangen.

Die erfindungsgemässe Vorrichtung, wird nach vorheriger Untersuchung des Patienten, über Pharynx und Ösophagus in den Ventrikulus (Magen) geführt. Gegebenenfalls mittels Beobachtung über einen Röntgenschirm oder über Markierungsfelder kann das Kissen im Magen gezielt nahe dem Fundus oder nahe dem Pylorus abgelegt werden. Anschliessend wird das Kissen über die Innensonde von deren distalem Ende her mit dem erwünschten Fluid, z.B. Wasser und/oder Gas, wie Luft, gegebenenfalls mit einem Indikator gemischt, gefüllt. Treten während des Füllvorgangs Beschwerden beim Patienten auf, etwa weil das Füllvolumen im Verhältnis zur Magengrösse zu gross gewählt worden ist, kann das Kissen ohne weiteres ganz oder teilweise entleert werden und kann gegebenenfalls auch wieder extrahiert werden. Wird das jeweilige Füllvolumen toleriert, werden die Kupplungsglieder am distalen Ende von Innensonde und Aussensonde ausser Eingriff gebracht und wird die Innensonde so weit in der Aussensonde zurückgezogen, wie es die Verbindung zwischen ihnen ermöglicht oder wie es aufgrund der Markierungsfelder zweckmässig erscheint. Durch das Zurückziehen der Innensonde bei unveränderter Lage der Aussensonde wird zunächst der Verschlusskörper in den Ring gezogen und wird durch Festsetzen in diesem das gefüllte Kissen verschlossen. Anschliessend wird entweder die Fadenschleife freigegeben oder kommen die Hakenelemente ausser Eingriff mit der zugehörigen Nut. Dadurch werden Applikator und Kissen voneinander getrennt. Der Applikator wird dann extrahiert, die Plazierung des Kissens ist beendet.

Gelangt das Kissen mit dem Applikator beim Einführen versehentlich in die Trachea, so kann die Erstickungssituation durch sofortiges Zurückziehen des Applikators risikolos beseitigt werden, da ein Abgleiten des Kissens vom Applikator sicher verhindert ist. Durch die distalseitigen Kupplungsglieder wird weiter sicher verhindert, dass versehentlich nur die Innensonde zurückgezogen wird, wodurch die Verbindung zwischen Kissen und Applikator getrennt werden könnte.

Wenn das Kissen im Magen undicht wird oder geplatzt ist, beispielsweise weil die Magensäfte den Werkstoff des Kissens zu stark angegriffen haben, kann dieser Zustand vom Patienten durch den beigefügten Indikator festgestellt werden. Beispielsweise tritt Methylenfarbstoff aus dem defekten Kissen aus, wird über den Darmtrakt absorbiert und mit dem Urin ausgeschieden, der dann blau gefärbt ist. Diese Anzeige ist jedoch nicht notwendig, da das Kissen komplikationslos mit dem Stuhl abgehen wird.

Wie erwähnt hat die Erfindung zwei wesentliche Vorteile. Zum einen ist ein Abrutschen des Kissens vom Applikator nicht möglich, da er mittels entweder der Fadenschlaufe 10, 49 oder über die in die Nut 37 eingreifenden Hakenelemente 39 fest verbunden ist. Die Trennung von Applikator und Kissen ist nur durch einen bewusst auszuführenden und nicht versehentlichen Handgriff möglich. Des weiteren ist eine bauliche Einheit gegeben, die bereits bei der Herstellung gebildet werden kann, bei der die gesamte Vorrichtung hergestellt wird, beispielsweise auch in eine sterile Aufreisspackung eingebracht wird, und in der Praxis dann sehr schnell zur Verfügung steht. Der Eingriff, d.h. das Plazieren des Kissens kann schnell und ambulant erfolgen, wobei das sichere dichte Verschliessen des Kissens nach Füllen mit dem Fluid gewährleistet ist.

Weiter kann in das Kissen ein pulverförmiges oder flüssiges keimtötendes Mittel, wie ein Antibiotikum, ein Chemotherapeutikum, ein Desinfiziens oder dergleichen, eingefüllt sein, das sich zweckmässigerweise beim Füllen des Kissens mit dem Fluid, hier vorzugsweise einem flüssigen Fluid, in diesem auflöst, wodurch das Wachstum von möglicherweise schädlichen Bakterien verhindert wird.

Es kann nämlich nicht zuverlässig verhindert werden, dass in das Kissen Bakterien eingeschleppt werden, die sich sonst bei der Körperwärme in dem Fluid vermehren und beim Platzen des Kissens den Intestinaltrakt (Darmtrakt) in grosser Anzahl überschwemmen würden, was, je nach Schädlichkeit der Bakterien, sehr unangenehme Folgen haben könnte.

Wie erwähnt, ist es wesentlich, dass eine sichere Verbindung zwischen Applikator und Kissen erreicht wird. Bezüglich des Ausführungsbeispiels gemäss den Fig. 5 und 6 sind noch weitere Ausführungsformen möglich, die im folgenden anhand der Fig. 9 bis 11 näher erläutert werden.

Bei der Ausführungsform gemäss Fig. 9 sind am proximalen Ende der Aussensonde 78 des Applikators 76 hakenförmige Elemente 75 als Eingriffsglieder dadurch gebildet, dass kugelförmige Elemente 71 über eine fadenartige Verbindung 79 mit dem proximalen Ende der Aussensonde 78 verbunden sind, die durch die Innensonde 77 des Applikators 76 in die Ringnut 74 des Rings 73 des Kissens 72 hineingedrückt werden. Wird die Innensonde 77 herausgezogen, sind die hakenförmigen Elemente 75 aufgrund ihrer fadenförmigen Verbindung 79 leicht verformbar und können durch leichtes Ziehen am Applikator 76 aus der Ringnut 74 einfach entfernt werden, wodurch das Kissen 72 vom Applikator 76 gelöst wird.

Fig. 10 zeigt, dass es nicht notwendigerweise erforderlich ist, eine Ringnut in dem Ring 83 vorzusehen und die hakenförmigen Elemente an der Aussensonde anzubringen. Bei der Ausführungsform gemäss Fig. 10 weist die Aussensonde 88 des Applikators 86 an ihrem proximalen Ende eine innenliegende Ringnut 84 auf und sind am im Kissen 82 befestigten Ring 83 an dessen distalen Ende hakenförmige Elemente 85 angebracht, die, wie bei der Ausführungsform gemäss Fig. 9, aus einem kugelartigen Element 81 und einer fadenartigen Verbindung 89 bestehen. Wie bei der Ausführungsform gemäss Fig. 9, drückt die Innensonde 87 des Applikators 86 die kugelartigen Elemente 81 in die Ringnut 84 und erreicht dadurch die sichere Verbindung zwischen Applikator 86 und Kissen 82. Bei Herausziehen der Innensonde 87 ist der Eingriff zwischen der Nut 84 und dem kugelartigen Element 81 durch leichtes Ziehen am Applikator 86 lösbar.

Die Ausführungsform gemäss Fig. 11 ähnelt der gemäss Fig. 5 darin, dass hakenförmige Elemente 95 vorgesehen sind. Diese sind jedoch, ähnlich wie bei der Ausführungsform gemäss Fig. 10, am distalen Ende des Rings 93 des Kissens 92 angebracht, insbesondere angeformt und können, wie bei der Ausführungsform gemäss Fig. 5, eine radial nach innen wirkende Vorspannung besitzen. Die hakenförmigen Elemente 95 werden durch die Innensonde 97 des Applikators 96 in eine Ringnut 94 im proximalen Ende der Aussensonde 98 des Applikators 96 hineingedrückt. Wenn die Innensonde 97 herausgezogen ist, wird in der anhand Fig. 5 und 6 erläuterten Weise der Eingriff zwischen der Nut 94 und den hakenförmigen Elementen 95 gelöst und sind Kissen 92 und Applikator 96 voneinander getrennt.

Selbstverständlich sind noch andere Ausführungsbeispiele möglich.

**Patentansprüche**

1. Vorrichtung zum Verkleinern des Magenreservoirs mittels eines intragastral applizierbaren Kissens (2, 25, 42, 62, 72, 82, 92) aus einem magenfreundlichen Werkstoff, das eine ventilartig verschliessbare Öffnung (4, 29, 44) aufweist und mit einem peroral einführbaren Applikator (6, 26, 46, 66, 76, 86, 96) lösbar verbunden ist, der aus einer Aussensonde (8, 28, 48, 68, 78, 88, 98) und einer darin geführten Innensonde (7, 27, 47, 67, 77, 87, 97) besteht, wobei beim Plazieren des Kissens die Innensonde mit dem Kissen in Eingriff steht und dabei das Innere des Kissens über die Innensonde und die ventilartig verschliessbare Öffnung mit einem magen- und darmverträglichen Fluid füllbar ist und zum Lösen des Kissens vom Applikator die Innensonde in Extrahierrichtung gegenüber der Aussensonde bewegt wird und hierbei die Innensonde ausser Eingriff mit dem Kissen kommt, dadurch gekennzeichnet, dass die Innensonde (7, 27, 47, 67, 77, 87, 97) durch die ventilartig verschliessbare Öffnung (4, 29, 44) in das Innere des Kissens (2, 25, 42, 62, 72, 82, 92) verläuft und zwischen Aussensonde (8, 28, 48, 68, 78, 88, 98) und Kissen (2, 25, 42, 62, 72, 82, 92) Eingriffsteile (10, 49, 69) vorgesehen sind, die ausser Eingriff kommen, wenn die Innensonde (7, 27, 47, 67, 77, 87, 97) bei ihrer Bewegung in Extrahierrichtung aus der ventilartig verschliessbaren Öffnung (4, 29, 44) herausgezogen ist.

2. Vorrichtung nach Anspruch 1, dadurch gekennzeichnet, dass das eine Eingriffsteil eine an Kissen (2, 42, 62) oder Aussensonde (8, 48, 68) befestigte Fadenschleife (10, 49, 69) ist, die zum Eingriff durch eine Öffnung (11, 50, 64) nahe dem proximalen Ende der Aussensonde (8, 48) bzw. in einem Teil (Ring 65) des Kissens (62) geführt ist und die von der Innensonde (7, 47, 67) durchgriffen wird.

3. Vorrichtung nach Anspruch 1, dadurch gekennzeichnet, dass das eine Eingriffsteil durch eine Nut (37, 74, 84, 94) im Bereich der Öffnung (29) des Kissens (25, 72) oder im proximalen Abschnitt der Aussensonde (88, 98) gebildet ist, dass das andere Eingriffsteil durch am proximalen Ende (38) der Aussensonde (28) bzw. dem gegenüberliegenden Bereich der Öffnung des Kissens (82, 92) angebrachte, verschwenkbare Hakenelemente (39, 75, 85, 95) gebildet ist und dass zum Eingriff die die Öffnung (29) durchsetzende Innensonde (27, 77, 87, 97) die Hakenelemente (39, 75, 85, 95) in die Nut (37, 74, 84, 94) drückt.

4. Vorrichtung nach Anspruch 3, dadurch gekennzeichnet, dass die Hakenelemente (39, 95) am proximalen Ende (38) der Aussensonde (28) bzw. im Bereich der Öffnung des Kissens (92) dort (Ring 95) angeformt sind.

5. Vorrichtung nach einem der Ansprüche 1 bis 4, dadurch gekennzeichnet, dass in der Öffnung (4, 29, 44) des Kissens (2, 25, 42, 62, 72, 82, 92) ein Ring (5, 30, 45, 65, 75, 85, 95) mit Mittelöffnung (9, 31) befestigt ist, durch die die Innensonde (7, 27, 47) in das das Innere des Kissens (2, 25, 42, 62, 72, 82, 92) ragt und die durch Herausziehen der Innensonde (7, 27, 47, 67, 77, 87, 97) verschliessbar ist.

6. Vorrichtung nach Anspruch 5 und nach Anspruch 3 oder 4, dadurch gekennzeichnet, dass die Nut (37, 74) in dem Ring (30, 73) ausgebildet ist.

7. Vorrichtung nach Anspruch 5, dadurch gekennzeichnet, dass zumindest der die Mittelöffnung (9) umgebende Bereich des Ringes (5) derart elastisch ausgebildet ist, dass die Mittelöffnung (9) nach Herausziehen der Innensonde (7) zumindest bis zu einem vorgegebenen Fluiddruck nicht verschlossen ist.

8. Vorrichtung zum Verkleinern des Magenreservoirs mittels eines intragastral applizierbaren Kissens (2, 25, 42, 62, 72, 82, 92) aus einem magenfreundlichen Werkstoff, das eine ventilartig verschliessbare Öffnung (4, 29, 44) aufweist und mit einem peroral einführbaren Applikator (6, 26, 46, 66, 76, 86, 96) lösbar verbunden ist, der aus einer Aussensonde (8, 28, 48, 68, 78, 88, 98) und einer darin geführten Innensonde (7, 27, 47, 67, 77, 87, 97) besteht, wobei beim Plazieren des Kissens die Innensonde mit dem Kissen in Eingriff steht und dabei das Innere des Kissens über die Innensonde und die ventilartig verschliessbare Öffnung mit einem magen- und darmverträglichen Fluid füllbar ist und zum Lösen des Kissens vom Applikator die Innensonde in Extrahierrichtung gegenüber der Aussensonde bewegt wird und hierbei die Innensonde ausser Eingriff mit dem Kissen kommt, dadurch gekennzeichnet, dass die ventilartig verschliessbare Öffnung (4, 29, 44) einen Ring (5, 30, 45) mit Mittelöffnungen (9, 31) aufweist, durch die die Innensonde (7, 27, 47) in das Innere des Kissens (2, 25, 42) verläuft, mindestens der die Mittelöffnung (9, 31) umgebende Bereich des Rings (5, 30, 45) elastisch ausgebildet ist und die Innensonde (7, 27, 47) an ihrem Vorderende (19) einen Verschlusskörper (16, 36, 51) trägt, der beim Herausziehen der Innensonde (7, 27, 47) aus der Mittelöffnung (9, 31) in diese eingesetzt wird und diese fluiddicht verschliesst.

9. Vorrichtung nach Anspruch 8, dadurch gekennzeichnet, dass der Ring (30) aus unverformbarem Kunststoff besteht und in seiner Mittelöffnung (31) einen in eine Ringnut (32) einsetzbaren O-Ring (33) aus elastischem Werkstoff aufweist.

10. Vorrichtung nach Anspruch 8 oder 9, dadurch gekennzeichnet, dass in den proximalen Abschnitt (19) der Innensonde (7) der Verschlusskörper (16) über einen Halsabschnitt (17) eingesteckt ist und dass die Innensonde (7) in Anschluss an den proximalen Abschnitt (19) und höchstens über den Bereich, der sich beim Plazieren innerhalb des Kissens (2) befindet, Perforationen (22) für die Fluidfüllung des Kissens (2) aufweist.

11. Vorrichtung nach Anspruch 10, dadurch gekennzeichnet, dass der proximale Abschnitt (19) mittels einer Zwischenwand (20) abgetrennt ist.

12. Vorrichtung nach Anspruch 10 oder 11, dadurch gekennzeichnet, dass der Aussendurchmesser des Halsabschnitts (17) des Verschlusskörpers (16) grösser als der Innendurchmesser des unverformten elastischen Bereichs des Rings (5) ist und dass der Aussendurchmesser des Halsabschnitts (17) des Verschlusskörpers (16) etwas grösser als der oder gleich dem Aussendurchmesser der Innensonde (7) ist.

13. Vorrichtung nach Anspruch 10 oder 12, dadurch gekennzeichnet, dass der Verschlusskörper (16, 36, 51) einen an den Halsabschnitt (17, 34) angeformten verdickten Abschnitt (18, 35) aufweist.

14. Vorrichtung nach einem der Ansprüche 1 bis 13, dadurch gekennzeichnet, dass die Innensonde (7, 47) und die Aussensonde (8, 48) am distalen Ende fest aber lösbar miteinander verbunden sind.

15. Vorrichtung nach Anspruch 14, gekennzeichnet, durch eine Schnellkupplung, wie eine Luer-Lock-Verschraubung.

16. Vorrichtung nach einem der Ansprüche 1 bis 15, gekennzeichnet, durch eine Erfassungseinrichtung (15, 49, 60, 61) am distalen Ende des Applikators (6, 46) zum Erfassen des Ausmasses der Bewegung zwischen Innensonde (7, 47) und Aussensonde (8, 48).

17. Vorrichtung nach Anspruch 16, dadurch gekennzeichnet, dass die Erfassungseinrichtung eine verformbare, jedoch nicht in Längsrichtung dehnbare Verbindung (15, 59) zwischen Innensonde (7, 47) und Aussensonde (8, 48) an deren distalen Enden aufweist, deren Länge mindestens dem zum Lösen des Kissens (2, 42) vom Applikator (6, 46) erforderlichen Bewegungsweg entspricht.

18. Vorrichtung nach einem der Ansprüche 1 bis 17, dadurch gekennzeichnet, dass am proximalen Endabschnitt der Aussensonde (48) eine das Kissen (42) zumindest im wesentlichen über seine Länge umgebende schlauchartige Hülle (53) angebracht ist, die nach dem Plazieren mit dem Applikator (46) auftrennbar und mit diesem entfernbar ist.

19. Vorrichtung nach einem der Ansprüche 1 bis 18, dadurch gekennzeichnet, dass mindestens die Reibungsbereiche zwischen Kissen (2, 25, 42) und Applikator (6, 26, 46) und/oder Kissen (42) und Hülle (53) und/oder Verschlusskörper (16, 36, 51) und Innensonde (7, 27, 47) mit einem Schmier- und Dichtungsmittel, wie Silikonöl beschichtet sind.

20. Vorrichtung nach einem der Ansprüche 1 bis 19, dadurch gekennzeichnet, dass zumindest der Applikator (6, 26, 36), vorzugsweise auch der Ring (5, 30, 45) und/oder Verschlusskörper (16, 36, 51) aus einem Werkstoff bestehen, der röntgenstrahlendurchlässiger ist als beim Plazieren umgebendes Gewebe.

21. Vorrichtung nach einem der Ansprüche 1 bis 20, dadurch gekennzeichnet, dass der Applikator am distalen Endabschnitt flexibler ist als am proximalen Endabschnitt.

**Claims**

1. Device for reducing the stomach reservoir by means of an intragastrally applicable cushion (2, 24, 42, 62, 72, 82, 92) made of a material harmless to the stomach, which has an aperture (4, 29, 44) which can close like a valve, and is detachably connected to a perorally insertable applicator (6, 26, 46, 66, 76, 86, 96), which consists of an outer probe (8, 28, 48, 68, 78, 88, 98) and an inner probe (7, 27, 47, 67, 77, 87, 97) guided therein, the inner probe engaging with the cushion if the cushion bursts and thereby the interior of the cushion being able to fill with a fluid compatible with the stomach and intestine via the inner probe and the aperture that can be closed like a valve and to detach the cushion from the applicator, the inner probe is moved in the direction of extraction vis-a-vis the outer probe and hereby the inner probe disengaging with the cushion, characterised in that the inner probe (7, 27, 47, 67, 77, 87, 97) passes through the aperture (4, 29, 44) that can close like a valve into the interior of the cushion (2, 25, 42, 62, 72, 82, 92) and between the outer probe (8, 28, 48, 68, 78, 88, 98) and cushion (2, 25, 42, 62, 72, 82, 92) engagement parts (10, 49, 69) are provided, which disengage when the inner probe (7, 27, 47, 67, 77, 87, 97) is withdrawn from the aperture (4, 29, 44) that can be closed like a valve when the inner probe moves in the extraction direction.

2. Device according to Claim 1, characterised in that one engagement part is a loop of thread (10, 49, 69) fastened to cushion (2, 42, 62) or outer probe (8, 48, 68), which to engage is guided through an aperture (11, 50, 64) near the proximal end of the outer probe (8, 48) or in a part (ring 65) of the cushion (62) and which is penetrated by the inner probe (7, 47, 67).

3. Device according to claim 1, characterised in that one engagement part is formed by a groove (37, 74, 84, 94) in the vicinity of the aperture (29) of the cushion (25, 72) or in the proximal section of the outer probe (88, 98), that the other engagement part is formed by swingable hook elements (39, 75, 85, 95) mounted at the proximal end (38) of the outer probe (28) or the opposite region of the aperture of the cushion (82, 92) and in that for engagement purposes the inner probe (27, 77, 87, 97) penetrating the aperture (29) presses the hook elements (39, 75, 85, 95) into the groove (37, 74, 84, 94).

4. Device according to Claim 3, characterised in that the hook elements (39, 95) are moulded to the proximal end (38) of the outer probe (28) or in the vicinity of the aperture of the cushion (92) there (ring 95).

5. Device according to one of Claims 1 to 4, characterised in that in the aperture (4, 29, 44) of the cushion (2, 25, 42, 62, 72, 82, 92) is fixed a ring (5, 30, 45, 65, 75, 85, 95) having a central aperture (9, 31), through which the inner probe (7, 27, 47) projects into the interior of the cushion (2, 25, 42, 62, 72, 82, 92) and which can be closed by withdrawing the inner probe (7, 27, 47, 67, 77, 87, 97).

6. Device according to Claim 5 and Claim 3 or 4, characterised in that the groove (37, 74) is constructed in the ring (30, 73).

7. Device according to Claim 5, characterised in that at least the area of the ring (5) surrounding the central aperture (9) is constructed elastically so that the central aperture (9) is not closed after the withdrawal of the inner probe (7) at least until a prescribed fluid pressure is attained.

8. Device for reducing the stomach reservoir by means of an intragastrally applicable cushion (2, 25, 42, 62, 72, 82, 92) made of a material harmless to the stomach, which has an aperture (4, 29, 44) that can close like a valve and which is detachably connected to a perorally insertable applicator (6, 26, 46, 66, 76, 86, 96), which consists of an outer probe (8, 28, 48, 68, 78, 88, 98) and an inner probe (7, 27, 47, 67, 77, 87, 97) guided therein, the inner probe engaging with the cushion if the cushion bursts and thereby the interior of the cushion being able to be filled with fluid compatible with the stomach and the intestine via the inner probe and aperture that can close like a valve and to detach the cushion from the applicator the inner probe being moved in the extraction direction vis-a-vis the outer probe and hereby the inner probe disengaging with the cushion, characterised in that the aperture (4, 29, 44) that can close like a valve has a ring (5, 30, 45) having central apertures (9, 31), through which the inner probe (7, 27, 47) passes into the interior of the cushion (2, 25, 42), at least the region of the ring (5, 30, 45) surrounding the central aperture (9, 31) is constructed elastically and the inner probe (7, 27, 47) bears on its front end (19) a sealing component (16, 36, 51), which, when the inner probe (7, 27, 47) is withdrawn from the central aperture (9, 31), is inserted into said aperture and closes it in a fluid-tight manner.

9. Device according to Claim 8, characterised in that the ring consists of non-deformable plastic and in its central aperture (31) has an O-ring (33) that can be inserted into an annular groove (32) made of elastic material.

10. Device according to Claim 8 or 9, characterised in that the sealing component (16) is inserted into the proximal section (19) of the inner probe (7) via a throat section (17) and in that in contact with the proximal section (19) and at the most above the region inside the cushion (2) when it bursts the inner probe (7) has perforations (22) for filling the cushion (2) with fluid.

11. Device according to Claim 10, characterised in that the proximal section (19) is separated by means of a partition (20).

12. Device according to Claim 10 or 11, characterised in that the external diameter of the neck section (17) of the sealing component (16) is greater that the internal diameter of the non-deformed elastic region of the ring (5) and in that the external diameter of the throat section (17) of the sealing component (16) is somewhat greater than or equal to the external diameter of the inner probe (7).

13. Device according to Claim 10 or 12, characterised in that the sealing component (16, 36, 51) has a thickened section (18, 35) formed on the throat section (17, 34).

14. Device according to one of Claims 1 to 13, characterised in that the inner probe (7, 47) and the outer probe (8, 48) are connected to one another securely but detachably at the distal end.

15. Device according to Claim 14, characterised by a quick-disconnect coupling, such as a Luer lock coupling.

16. Device according to one of Claims 1 to 15, characterised by a detection appliance (15, 49, 60, 61) for detecting the amount of movement between the inner probe (7, 47) and the outer probe (8, 48).

17. Device according to Claim 16, characterised in that the detection appliance has a deformable connection (15, 59), which however is not extensible in the longitudinal direction, between the inner probe (7, 47) and the outer probe (8, 48) at its distal ends, the length of which corresponds at least to the path of movement required to detach the cushion (2, 42) from the applicator (6, 46).

18. Device according to one of Claims 1 to 17, characterised in that on the proximal end section of the outer probe (48) is mounted a hose-like casing (53) surrounding the cushion (42) at least substantially over its length, which can be ripped open after bursting with the applicator (46) and removed with this.

19. Device according to one of Claims 1 to 18, characterised in that at least the areas of friction between the cushion (2, 25, 42) and the applicator (6, 26, 46) and/or the cushion (42) and casing (53) and/or the sealing component (16, 36, 51) and inner probe (7, 27, 47) are coated with a lubricant and sealant, such as siliconale (?).

20. Device according to one of Claims 1 to 19, characterised in that at least the applicator (6, 26, 36), and preferably also the ring (5, 30, 45) and/or the sealing component (16, 36, 51) consist of a material which is more radiopaque than surrounding fabric when the cushion bursts.

21. Device according to one of Claims 1 to 20, characterised in that the applicator is more flexible at the distal end section than at the proximal end section.

**Revendications**

1. Dispositif pour la diminution du réservoir d'estomac par un coussin (2, 25, 42, 62, 72, 82, 92) mis en place par intubation gastrique et fabriqué d'un matériel gastrophile. Le coussin possède un orifice (4, 29, 44) se fermant à la manière d'une vanne et il est lié d'une façon détachable à un applicateur (6, 26, 46, 66, 76, 86, 96) introduisible per os. L'applicateur se compose d'une sonde externe (8, 28, 48, 68, 78, 88, 98) et d'une sonde interne (7, 27, 47, 67, 77, 87, 97) manipulée à son intérieur. Lors de la mise en place du coussin, la sonde interne est liée au coussin et ainsi l'intérieur du coussin peut être rempli à travers la sonde interne et l'orifice se fermant à la manière d'une vanne par un liquide gastro- et intestinophile. Le détachement du coussin du dispositif de la mise en place s'effectue en tirant la sonde interne, par rapport à la sonde externe, dans le sens de l'extraction.
Ce procédé est caractérisé par le fait que la sonde interne (7, 27, 47, 67, 77, 87, 97) évolue à travers l'orifice (4, 29, 44) se fermant à la manière d'une vanne jusqu'à l'intérieur du coussin (2, 25, 42, 62, 72, 82, 92) et qu'entre la sonde externe (8, 28, 48, 68, 78, 88, 98) et le coussin (2, 25, 42, 62, 72, 82, 92) sont prévue des dispositifs de liaison (10, 49, 69) qui perdent leur fonction quand la sonde interne (7, 27, 47, 67, 77, 87, 97), dans son mouvement en direction de l'extraction, est sortie de l'orifice (4, 29, 44) se fermant à la manière d'une vanne.

2. Dispositif selon revendication 1, caractérisé par le fait qu'un des éléments de fixation consiste en une boucle de fil (10, 49, 69), fixée au coussin (2, 42, 62) ou à la sonde externe (8, 48, 68) et que cette boucle de fil passe pour la fixation par un orifice (11, 50, 64) près de l'extrémité proximale de la sonde externe (8, 48) respectivement dans une partie (anneau 65) du coussin (62) et qui est fixée par la sonde interne (7, 47, 67).

3. Dispositif selon revendication 1, caractérisé par le fait qu'un des éléments de fixation est formé par une rainure (37, 74, 84, 94) dans la zone de l'orifice (29) du coussin (25, 72) ou dans la partie proximale de la sonde externe (88, 98) et que l'autre élément de fixation est constitué par un système de crochets pivotables, posé à l'extrémité proximale (38) de la sonde externe (28) respectivement dans l'espace vis-à-vis de l'orifice du coussin (82, 92) et que pour la fixation, la sonde interne (27, 77, 87, 97), qui traverse l'orifice (29), pousse le système des crochets (39, 75, 85, 95) dans la rainure (37, 74, 84, 94).

4. Dispositif selon revendication 3, caractérisé par le fait, que le système de crochets (39, 95) à l'extrémité proximale (38) de la sonde externe (28) respectivement dans l'espace de l'orifice du coussin (92) est à cet endroit (anneau 95) incorporé par moulage.

5. Dispositif selon une des revendications 1 à 4, caractérisé par le fait que dans l'orifice (4, 29, 44) du coussin (2, 25, 42, 62, 72, 82, 92) un anneau (5, 30, 45, 65, 75, 85, 95) avec une ouverture centrale (9, 31) est installé, à travers laquelle la sonde interne (7, 27, 47) se dresse dans l'intérieur du coussin (2, 25, 42, 62, 72, 82, 92) et qui peut être fermée lorsque l'on enlève la sonde interne (7, 27, 47, 67, 77, 87, 97).

6. Dispositif selon revendication 5 et selon revendication 3 ou 4, caractérisé par le fait que la rainure (37, 74) est formée dans l'anneau (30, 73).

7. Dispositif selon revendication 5, caractérisé par le fait qu'au moins la zone de l'anneau (5) qui entoure l'ouverture centrale (9) est fabriquée d'une manière qui donne une telle élasticité, que l'ouverture centrale (9), après l'éloignement de la sonde interne (7), n'est pas fermée, tout au moins jusqu'à une pression de liquide donnée.

8. Dispositif pour la diminution du réservoir d'estomac par un coussin (2, 25, 42, 62, 72, 82, 92) mis en place par intubation gastrique et fabriqué d'un matériel gastrophile possédant un orifice (4, 29, 44) se fermant à la manière d'une vanne et lié d'une façon détachable à un applicateur (6, 26, 46, 66, 76, 86, 96) introduisible per os qui consiste en une sonde externe (8, 28, 48, 68, 78, 88, 98) et une sonde interne (7, 27, 47, 67, 77, 87, 97) dirigée en son intérieur.
Lors de la mise en place du coussin, la sonde interne est liée au coussin et ainsi l'intérieur du coussin peut être rempli à travers la sonde interne et l'orifice se fermant à la manière d'une vanne, par un liquide gastro- et intestinophile.
Pour détacher le coussin de l'intérieur de la mise en place, on tire la sonde interne, par rapport à la sonde externe, dans le sens de l'extraction et on libère ainsi la sonde interne du coussin. Ceci est caractérisé par le fait que l'orifice (4, 29, 44) se fermant à la manière d'une vanne possède un anneau (5, 30, 45) avec une ouverture centrale (9, 31), à travers laquelle la sonde interne (7, 27, 47) s'élève dans l'intérieur du coussin (2, 25, 42), et qui est fabriqué d'une manière qu'au moins la zone de l'anneau (5, 30, 45) qui entoure l'ouverture centrale (9, 31) soit élastique et que la sonde interne (7, 27, 47) est équipée à son extrémité antérieure (19) d'un corps de fermeture (16, 36, 51) qui, lors de l'éloignement de la sonde interne (7, 27, 47) de l'ouverture centrale (9, 31) se met dans l'ouverture centrale et la rend étanche aux liquides.

9. Dispositif selon revendication 8, caractérisé par le fait que l'anneau (30) consiste en matière plastique non déformable et possède dans son ouverture centrale (31) une rainure circulaire (32) dans laquelle on peut insérer un O-anneau fabriqué dans un matériel élastique.

10. Dispositif selon revendication 8 ou 9, caractérisé par le fait que le corps de fermeture (16) est encastré dans la section goulot de la partie proximale (19) de la sonde interne (7) et que la sonde interne (7) dans la suite de la partie proximale (19) est tout au plus au-dessus de la zone qui, lors de la mise en place, se trouve à l'intérieur du coussin (2), possède des perforations (22) pour le remplissage du coussin (2) par le liquide.

11. Dispositif selon revendication 10, caractérisé par le fait que la partie proximale (19) est séparée par une cloison (20).

12. Dispositif selon revendication 10 ou 11, caractérisé par le fait que le diamètre extérieur de la section goulot (17) du corps de fermeture (16) est plus grand que le diamètre intérieur de la zone élastique et non déformée de l'anneau (5) et que le diamètre extérieur de la section goulot (17) du corps de fermeture (16) est légèrement plus grand que le diamètre extérieur de la sonde interne (7).

13. Dispositif selon revendication 10 ou 12, caractérisé par le fait que le corps de fermeture (16, 36, 51) possède une partie épaissie (18, 35), moulée à la section goulot.

14. Dispositif selon revendication 1 à 13, caractérisé par le fait que la sonde interne (7, 47) et la sonde externe (8, 48) sont fixées à l'extrémité distale d'une matière solide mais détachable.

15. Dispositif selon revendication 14, caractérisé par un embrayage rapide, comme un raccord du type Luer-Lock.

16. Dispositif selon une des revendications 1 à 15, caractérisé par un appareil d'enregistrement (15, 49, 60, 61) à l'extrémité distale de l'applicateur (6, 46) pour l'enregistrement de l'importance du mouvement entre la sonde interne (7, 47) et la sonde externe (8, 48).

17. Dispositif selon revendication 16, caractérisé par le fait que l'appareil d'enregistrement possède un lien déformable (15, 59) mais cependant pas extensible dans le sens longitudinal, entre la sonde interne (7, 47) et la sonde externe (8, 48) à son extrémité distale dont la longueur correspond au moins à l'importance du mouvement nécessaire pour détacher le coussin (2, 42) de l'applicateur (6, 46).

18. Dispositif selon une des revendications 1 à 17, caractérisé par le fait qu'à l'extrémité proximale de la sonde externe (48) est aménagé une gaine ajout la forme d'un tuyau qui couvre tout au moins l'essentiel de la longueur du coussin (42) et qui, après la mise en place avec l'applicateur, peut être décousue par l'applicateur et enlevée avec celui-ci.

19. Dispositif selon une des revendications 1 à 18, caractérisé par le fait qu'au moins la zone de friction entre le coussin (2, 25, 42) et l'applicateur (6, 26, 46) et/ou le coussin (42) et la gaine (53) et/ou le corps de fermeture (16, 36, 51) et la sonde interne (7, 27, 47) sont enduits d'un produit de lubrification et d'étanchéité, tel que l'huile de silicone.

20. Dispositif selon une des revendications 1 à 19, caractérisé par le fait qu'au moins l'applicateur (6, 26, 36), de préférence aussi l'anneau (5, 30, 45) et/ou le corps de fermeture (16, 36, 51) consistent d'un matériel qui est moins perméable aux rayons X que le tissu environnant lors de l'intubation.

21. Dispositif selon une des revendications 1 à 20, caractérisé par le fait que l'applicateur est plus flexible dans son extrémité distale qu'à son extrémité proximale.

Fig. 1

Fig. 2

Fig. 3

Fig. 4

Fig. 5

Fig. 6

*Fig. 7*

*Fig. 8*

*Fig. 9*

*Fig. 10*

*Fig. 11*